# EUROPÄISCHE PATENTSCHRIFT

(11) **EP 0 827 385 B1**
(45) Veröffentlichungstag und Bekanntmachung des Hinweises auf die Patenterteilung: **18.10.2000**
(21) Anmeldenummer: 96914958.2
(22) Anmeldetag: 19.04.1996
(51) Int. Cl.: A61B 17/72, A61F 2/36

(54) **NAGEL ZUR LAGE- UND FORMFIXIERUNG VON GEBROCHENEN RÖHRENKNOCHEN**
NAIL FOR MAINTAINING THE LOCATION AND SHAPE OF BROKEN LONG BONES
CLOU DESTINE A MAINTENIR LA POSITION ET LA FORME D'OS LONGS FRACTURES

(30) Priorität: 21.04.1995 DE 19514758
(43) Veröffentlichungstag der Anmeldung: 11.03.1998
(73) Patentinhaber: Werding, Gerd, 85049 Ingolstadt (DE); Schneider, Willi, 85049 Ingolstadt (DE)
(72) Erfinder: Werding, Gerd, 85049 Ingolstadt (DE); Schneider, Willi, 85049 Ingolstadt (DE)
(74) Vertreter: Kilian, Helmut, Dr.
(86) Internationale Anmeldenummer: EP9601652
(87) Internationale Veröffentlichungsnummer: WO9632899

(56) Entgegenhaltungen:
- DE-A- 2 821 785
- FR-A- 2 629 337
- FR-A- 2 674 119
- US-A- 4 313 434
- US-A- 5 102 413
- US-A- 5 376 123

## Beschreibung

Die Erfindung betrifft einen Nagel zur Lage- und Formfixierung von gebrochenen Röhrenknochen.

Bislang werden zur inneren Stabilisierung gebrochener langer Röhrenknochen vorwiegend im Querschnitt U- bzw. V-förmige starre relativ großdimensionierte Stahlnägel verwendet. Sie stabilisieren den Knochen nach dem Prinzip einer Dreipunktabstützung am Anfang, am Ende und im Mittelbereich des Nagels. Um derartige Nägel einzubringen, müssen großdimensionierte Kanäle durch die Oberfläche des Knochens und anschließend durch die Markhöhle gefräst werden, die dem Durchmesser des verwendeten implantierten Nagels entsprechen. Dies hat den Nachteil, daß zur Herstellung dieses Kanals fast der gesamte Markraum ausgefräst werden muß, wodurch insbesondere die Blutversorgung des Knochens verletzt wird. Durch die Dreipunktabstützung erfolgt überdies die Kraftübertragung auf eine verhältnismäßig kleine Fläche und zur Sicherung der Rotationsstabilität müssen zusätzliche Mechanismen, wie etwa Verriegelungsschrauben und dergleichen, verwendet werden.

Auch das Entfernen des Marknagels nach erfolgter Abheilung ist mit relativ großem Aufwand verbunden. Der in der Markhöhle verklemmte Nagel muß mit einem speziellen Ausschlaginstrumentarium mit verhältnismäßig großem Kraftaufwand aus der Markhöhle herausgeschlagen werden, wodurch wiederum eine erhebliche Schädigung des Markraumes bewirkt werden kann.

Aus DE-C-32 01 056 ist ein Marknagel bekannt, bei dem der Nagelschaft ein Hohlkörper aus einer Memory-Legierung ist, die in Abhängigkeit von der Temperatur jeweils einen von zwei möglichen Formzuständen einnimmt. Damit kann der Marknagel in situ aus einem kleinquerschnittigen in einen aufgeweiteten Zustand überführt werden und umgekehrt. Nachteilig an diesem bekannten Marknagel ist die thermische Belastung des Knochens und Knochenmarks, die die für die Aufweitung des Nagelschafts erforderliche Erwärmung mit sich bringt.

Ein Nagel gemäß Oberbegriff des Patentanspruchs 1 ist aus US-A-5 102 413 bekannt. Bei dem bekannten Nagel umgibt eine einzige Blase als Vergrößerungskörper den Grundkörper vollständig.

Aufgabe der Erfindung ist es, einen Nagel zur Lage- und Formfixierung von gebrochenen Röhrenknochen zu schaffen, welcher bei guter Stabilisierung ohne großräumige Verletzung der Markhöhle implantiert werden kann und auch keinerlei thermische Belastungen des Knochens und Knochenmarks zur Folge hat.

Diese Aufgabe wird erfindungsgemäß durch einen Nagel mit den Merkmalen des Patentanspruchs 1 gelöst.

Der erfindungsgemäße Nagel kann im unaufgeweiteten Zustand, also bei geringem Durchmesser durch einen relativ kleindimensionierten Corticaliskanal in die Markhöhle eingeschoben werden. Ein die Markhöhle großflächig verletzendes Ausfräsen ist dabei nicht erforderlich. Im vollständig implantierten Zustand des Nagels wird sein Querschnitt dann ohne Erwärmung im zur Stabilisierung des gebrochenen Knochens erforderlichen Ausmaß aufgeweitet. Die abstützenden Kräfte verteilen sich dann großflächig. Durch den Flächenschluß und die sich ergebende Anpassung an die vorgegebene Form des Markraumes ist auch Rotationsstabilität gegeben.

Bei gegebener Rückführbarkeit der Querschnittsvergrößerung, wie nach Anspruch 2, läßt sich das Implantat zur Entfernung nach Beendigung des Heilungsvorgangs besonders gewebeschonend wieder entfernen.

Weitere bevorzugte Ausgestaltungen der Erfindung sind Gegenstand der weiteren Unteransprüche.

Bevorzugte Ausführungsformen der Erfindung werden nun anhand der beigefügten Zeichnungen beschrieben. Auf diesen zeigt:
Fig. 1 eine Ausführungsform des erfindungsgemäßen Nagels im Längsschnitt,
Fig. 2 einen Querschnitt längs Linie B-B der Fig. 1,

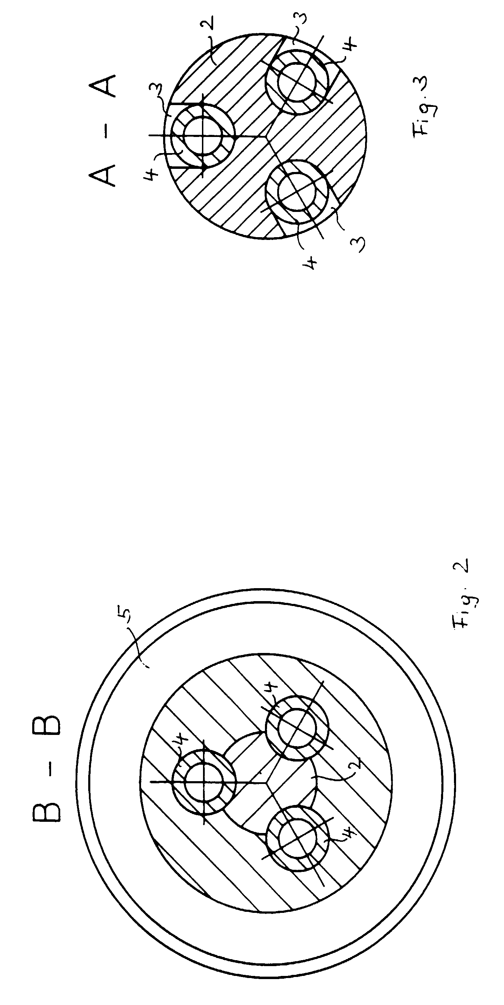

Fig. 3 ein Querschnitt längs Linie A-A der Fig. 1,
Fig. 4 nochmals einen Querschnitt gemäß Linie A-A bei eingezogenen und aufgeweiteten Vergrößerungskörpern,
Fig. 5 einen demjenigen der Fig. 4 entsprechenden Querschnitt bei einer anderen Ausführungsform von Schaftgrundkörper und Vergrößerungskörpern,
Fig. 6 einen demjenigen der Fig. 4 entsprechenden Querschnitt für eine weitere Ausführungsform von Schaftgrundkörper und Vergrößerungskörpern,
Fig. 7 eine Ausführungsform eines Ventils im Längsschnitt, eingesetzt in den Kopf des in Fig. 1 gezeigten Nagels,
Fig. 8 eine derjenigen der Fig. 7 entsprechende Ansicht für eine Ausführungsform, bei der der Nagelkopf als Teil des Ventils ausgebildet ist,

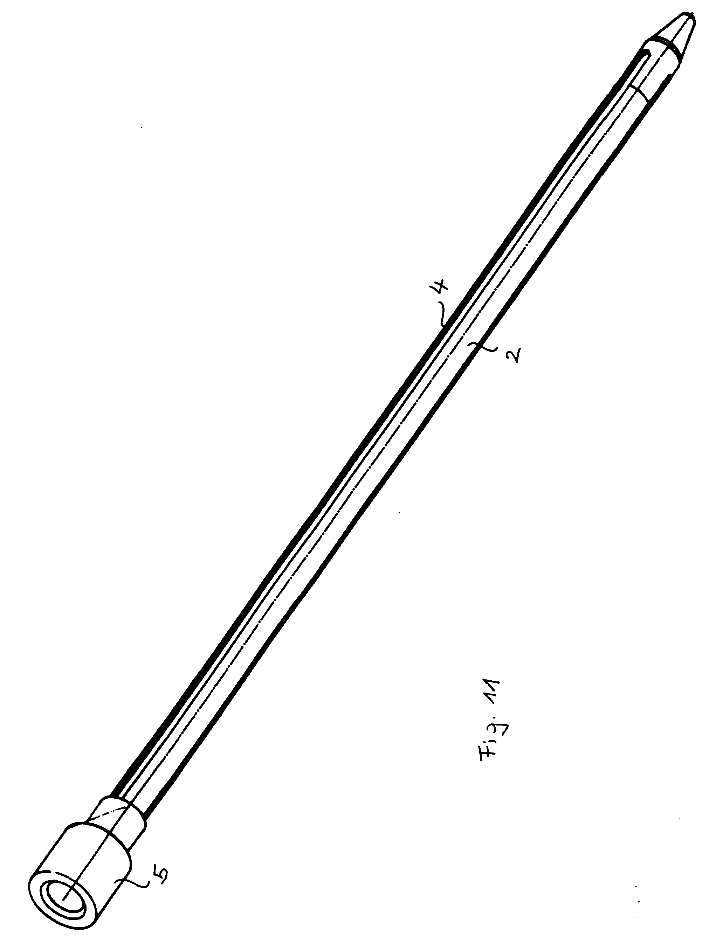

Fig. 9 eine Ausführungsform ähnlich derjenigen der Fig. 8 mit einer Kugel statt eines Kolbens als Verschlußkörper,
Fig. 10 eine Ausführungsform mit einer Durchstechmembran anstelle des Ventils,
Fig. 11 den Nagel der Fig. 1 im Schrägriß,
Fig. 12a und 12b einen erfindungsgemäßen Nagel im Längsschnitt bzw. abgebrochener Seitenansicht in einer Ausführungsform mit Schraubspitze, und
Fig. 13a und 13b eine Seitenansicht und eine Vorderansicht, von der Spitze her gesehen, eines Nagels mit einer Spreitzvorrichtung an der Spitze.

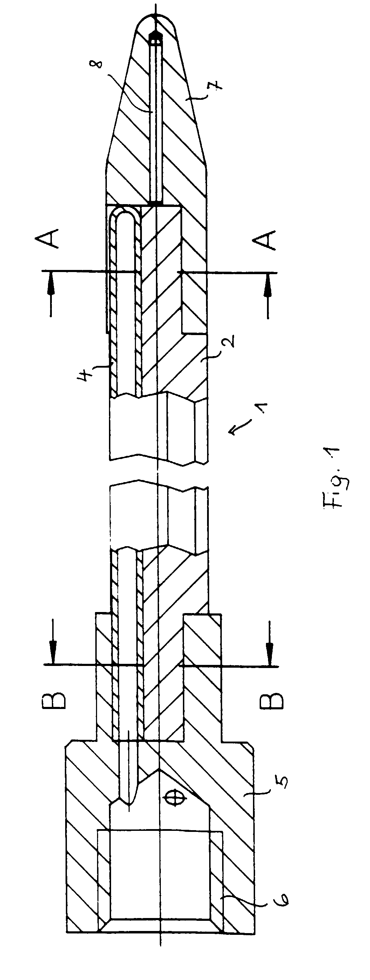

Der in Fig. 1 gezeigte Nagel für Röhrenknochen weist einen Schaft 1 mit einem Grundkörper 2 vorzugsweise aus gewebeverträglichem Kunststoff auf. Dieser im wesentlichen formstabile, aber bevorzugt mit einer gewissen Biegeelastizität ausgestattete Grundkörper 2, der in dieser Ausführungsform kreisförmigen Querschnitt hat, ist mit hier drei jeweils in Umfangsrichtung um 120° versetzten, in Längsrichtung verlaufenden Nuten 3 versehen, in denen im Querschnitt, vorzugsweise elastisch, dehnbare schlauchartige Vergrößerungskörper 4, ebenfalls vorzugsweise aus gewebeverträglichem Kunststoffmaterial, aufgenommen sind. Im unbelasteten Ruhezustand ragen die Vergrößerungskörper 4 über die Außenkontur des Grundkörpers 2 vorzugsweise nicht hinaus. Der Kopf 5 des Nagels ist als Anschlußteil für ein Füll- und Entlastungsventil, wie es in Fig. 10 gezeigt ist, ausgebildet und mit einem entsprechenden Anschlußgewinde 6 für das Ventil versehen. An der Spitze des Nagels befindet sich eine Endkappe 7 die zur Erleichterung des Einführens des Nagels vorzugsweise konisch geformt ist. Innerhalb der Spitze befindet sich vorzugsweise ein Metallstift 8, der unter Röntgenkontrolle sichtbar ist und damit das Einführen des Nagels erleichtert. Ebenfalls denkbar ist ein sich über die gesamte Länge des Nagels erstreckender Metallstreifen.

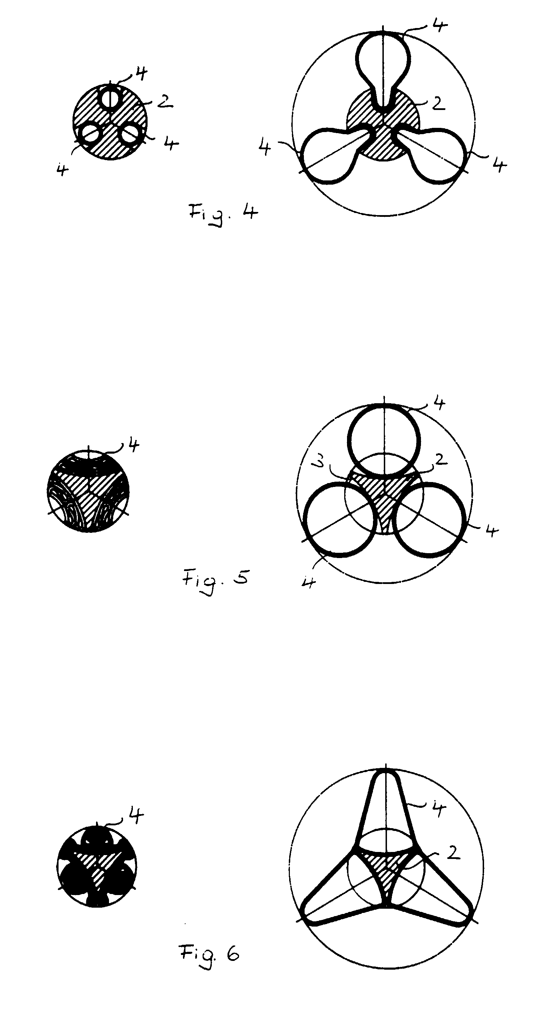

Werden die jeweils eine Kammer bildenden Vergrößerungskörper 4 durch Einpumpen eines Gases oder einer Flüssigkeit - physiologische Kochsalzlösung ist unter medizinischen Gesichtpunkten am geeignetsten - von innen unter Druck gesetzt, so weiten sich die Vergrößerungskörper 4 wie in Fig. 4 gezeigt auf, so daß der Querschnitt des Nagelschaftes 1 sich insgesamt vergrößert. Es ergibt sich ein im Querschnitt etwa sternartiges Gebilde. Die am weitesten nach außen ragenden Teile beschränken sich auf einen Bruchteil des Umkreisquerschnitts, so daß genügend Ausweichraum für die Verlagerung des Knochenmarkes verbleibt. Durch entsprechende Gestaltung des Querschnitts der Vergrößerungskörper läßt sich die Berührfläche zum Knochen nach Art und Größe beeinflussen. Die Biegeelastizität des Grundkörpers 2 und damit des Schafts 1 allgemein läßt den Schaft auch Krümmungen der Markhöhle folgen und sorgt zusammen mit der Natur der Vergrößerungskörper für eine über die Länge hinweg gleichmäßige Anlage am Knochen.

Fig. 5 zeigt eine Ausführungsform, bei der die kammerartigen Vergrößerungskörper nicht als Dehn-, sondern als Faltkörper ausgebildet sind, die im unbelasteten Zustand in den hier dann kehlenförmig ausgebildeten Nuten 3 liegen.

Fig. 6 zeigt eine Ausführungsform, bei welcher sich im unbelasteten Zustand gefaltete Vergrößerungskörper zu an den Spitzen gerundeten Dreiecksquerschnittsformen aufweiten.

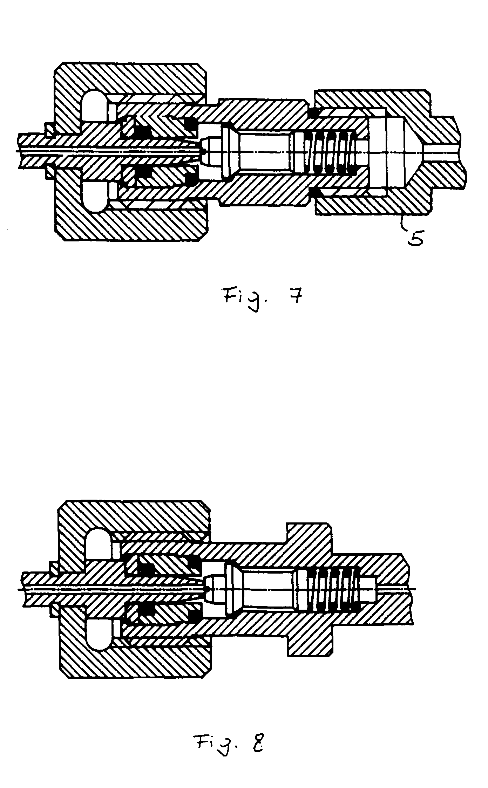

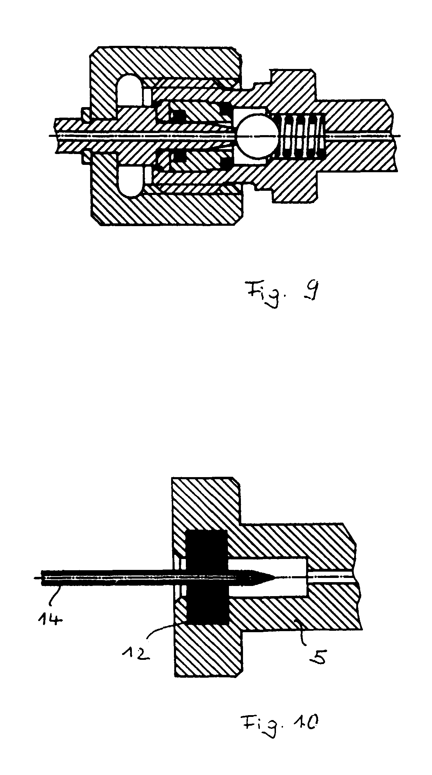

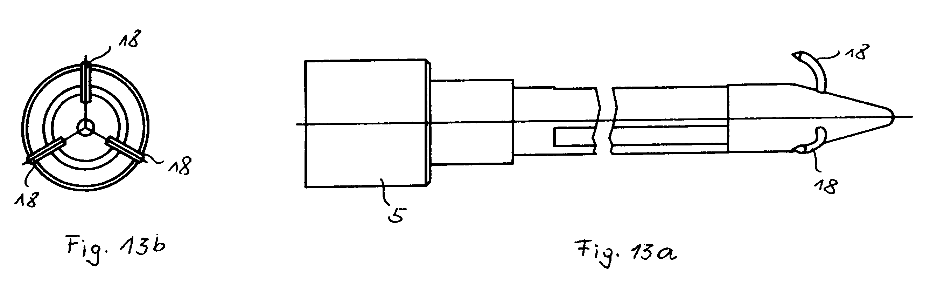

Zum Aufweiten des Nagelschaftes wird beispielsweise ein in den Nagelkopf 5 eingesetztes Ventil, wie es etwa in Fig. 7 gezeigt ist, verwendet. Bei den Ausführungsformen der Fig. 8 und 12 ist der Nagelkopf 5 so ausgebildet, daß er selbst Teil des Ventils ist. Das gleiche Ventil dient auch der Entlastung des oder der Vergrößerungskörper 4, also dem Abführen des eingefüllten Aufweitmediums.

Bei einer besonders bevorzugten Ausführungsform, wie sie in Fig. 10 dargestellt ist, befindet sich im Nagelkopf 5 lediglich eine Durchstechmembran 12 für eine Kanüle 14, mit der Pumpflüssigkeit in die Vergrößerungskörper eingefüllt werden kann. Nach dem Aufweitvorgang wird die Kanüle 14 wieder zurückgezogen, wobei sich die Durchstechmembran 12 von selbst schließt. Zum Abziehen der Pumpflüssigkeit nach Beendigung des Heilvorgangs wird die Kanüle erneut eingestochen und über sie die Flüssigkeit wieder abgezogen.

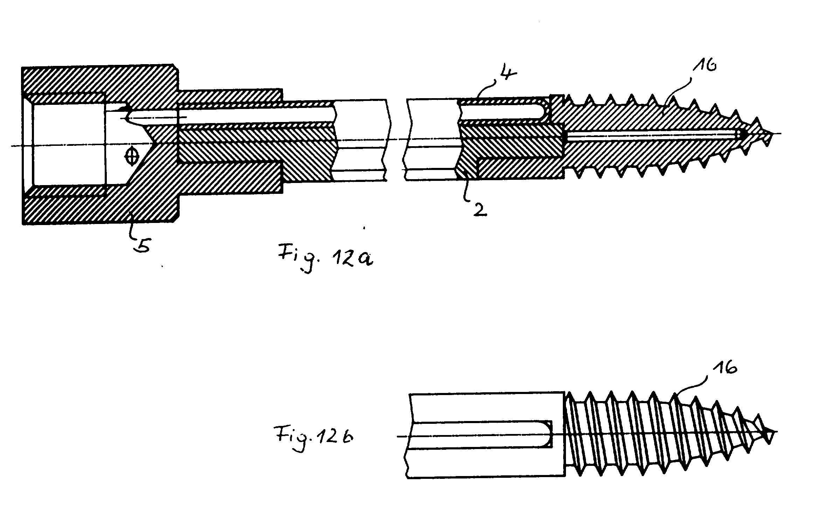

Fig. 11 zeigt den in Fig. 1 im Schnitt dargestellten Nagel noch einmal im Schrägriß. Eine typische Länge, entsprechend etwa der Länge des Oberschenkelknochens beträgt zwischen 25 und 35 cm.

Fig. 12a und 12b zeigt einen Nagelausführungsform mit einer Gewindespitze 16, die eine besondere Verankerung des Nagels im Kochen ebenso ermöglicht wie die Spreizvorichtung 18 der in den Fig. 13a und 13b gezeigten Nagelausführungsform.

In einer bevorzugten Ausgestaltung wird als Material für den Nagel körperresorbierbares Material verwendet. Damit erübrigt sich das Herausnehmen des Nagels nach erfolgter Heilung.

## Patentansprüche

1. Nagel zur Lage- und Formfixierung von gebrochenen Röhrenknochen, wobei der Nagel einen in situ im Querschnitt aufweitbaren Schatt (1) mit einem zentralen Grundkörper (2) und einem an dem Grundkörper angebrachten über seine Länge sich erstreckenden, kammerartigen Vergrößerungskörper (4) aufweist, der in situ durch Flüssigkeit oder Gas unter radialer Aufweitung desselben von innen unter Druck setzbar ist, dadurch gekennzeichnet, daß mehrere über die Länge des Grundkörpers (2) sich erstreckende Vergrößerungskörper (4) vorgesehen sind, welche um den Grundkörper (2) herum verteilt angeordnet sind.

2. Nagel nach Anspruch 1, dadurch gekennzeichnet, daß die Vergrößerungskörper (4) in situ in den unaufgeweiteten Zustand zurückführbar sind.

3. Nagel nach Anspruch 1 oder 2, dadurch gekennzeichnet, daß der Nagel am Kopfende ein Ventil zum Einfüllen und Ablassen von Flüssigkeit oder Gas zur Aufweitung bzw. Querschnittsverminderung des Nagelschaftes aufweist.

4. Nagel nach Anspruch 1 oder 2, dadurch gekennzeichnet, daß der Nagel am Kopfende eine Durchstechmembran (12) zum Einführen einer Kanüle (14) für das Einführen bzw. Ablassen von Flüssigkeit oder Gas aufweist.

5. Nagel nach einem der vorstehenden Ansprüche, dadurch gekennzeichnet, daß der Nagel eine Schraubspitze (16) aufweist.

6. Nagel nach einem der Ansprüche 1 bis 4, dadurch gekennzeichnet, daß der Nagel eine Spitze mit einer Aufspreizvorrichtung (18) aufweist.

## Claims

1. A nail for fixing the position and shape of broken long bones, said nail comprising a shank (1) expandable in cross section while in situ and having a central main body (2) and a chamber-like expansion element (4) attached to and extending over the length of the main body, which expansion element can be internally pressurized in situ and caused to expand radially by means of a gas of liquid, characterized in that several expansion elements (4) running the length of the main body (2) are provided spaced around the main body.

2. A nail according to Claim 1, characterized in that the expansion elements (4) can be returned to the non-expanded state while in situ.

3. A nail according to Claim 1 and 2, characterized in that the nail comprises at its head end a valve for introducing and discharging a liquid or gas to expand or reduce the cross sectional dimension of the nail shank.

4. A nail according to Claim 1 or 2, characterized in that the nail comprises at its head end a perforable membrane (12) for inserting a canula (14) to introduce or discharge a liquid or gas.

5. A nail according to any of the preceding Claims, characterized in that the nail comprises a screw tip (16).

6. A nail according to any of the Claims 1 to 4, characterized in that the nail comprises a tip with a strut arrangement (18).

## Revendications

1. Clou destiné à maintenir la position et la forme d'os longs fracturés, le clou présentant une tige (1), pouvant être élargie in situ au niveau de sa section transversale, avec un corps de base (2) central, et un corps d'agrandissement (4) du genre d'une chambre, monté sur le corps de base, s'étendant sur sa longueur, corps d'agrandissement qui est susceptible d'être placé sous pression depuis l'intérieur, in situ, par du liquide ou du gaz, en produisant un élargissement radial de celui-ci, caractérisé en ce que sont prévus plusieurs corps d'agrandissement (4) s'étendant sur la longueur du corps de base (2), répartis autour du corps de base (2).

2. Clou selon la revendication 1, caractérisé en ce que le corps d'agrandissement est rétractible in situ à l'état non élargi.

3. Clou selon la revendication 1 ou 2, caractérisé en ce que le clou présente à l'extrémité tête une soupape pour introduire et évacuer du liquide ou du gaz, dans le but d'agrandir ou de diminuer la section transversale de la tige du clou.

4. Clou selon la revendication 1 ou 2, caractérisé en ce que le clou présente à l'extrémité tête une membrane à transpercement (12) pour l'instruction d'une canule (14), pour effectuer l'introduction ou l'évacuation de liquide ou de gaz.

5. Clou selon l'une des revendications précédentes, caractérisé en ce que le clou présente une pointe de vis (16).

6. Clou selon l'une des revendications 1 à 4, caractérisé en ce que le clou présente une pointe dotée d'un dispositif d'écartement (18).
